# EUROPEAN PATENT APPLICATION

(11) **EP 2 233 088 A2**
(43) Date of publication of application: **29.09.2010**
(21) Application number: 10250550.0
(22) Date of filing: 23.03.2010
(51) Int. Cl.: A61B 17/34, A61B 17/04

(54) **Suture management system for surgical portal apparatus including slotted ring**

(30) Priority: 24.03.2009 US 162756 P; 22.02.2010 US 709593
(71) Applicant: Tyco Healthcare Group LP, North Haven, CT 06473 (US)
(72) Inventor: Taylor, Eric, East Hampton, CT 06424 (US)
(74) Representative: Soames, Candida Jane

(57) **Abstract**

A surgical portal apparatus for use in surgical procedures incorporating at least one suture is provided. The surgical portal apparatus includes a portal member defining a longitudinal axis and having a longitudinal opening therethrough for receiving a surgical object, and a suture retaining insert configured to be selectively received in the portal member. The suture retaining insert may include a base and a plurality of spaced projections extending radially outward from the base, the projections being radially spaced whereby adjacent projections define a suture receiving passage adapted to accommodate a suture.

## Description

### CROSS REFERENCE TO RELATED APPLICATION

The present application claims the benefit of and priority to U.S. Provisional Application Serial No. 61/162,756 filed on March 24, 2009, the entire contents of which are incorporated herein by reference.

### BACKGROUND

### Technical Field

The present disclosure relates to trocars and other surgical portal apparatus, and more particularly, relates to a suture management system for surgical portal apparatus that includes a slotted ring.

### Background of Related Art

Trocars and other surgical portal apparatus are known, as are myriad procedures that may be preformed using such assemblies. Many of the minimally invasive procedures performed through access assemblies necessitate or are simplified by the use of one or more sutures passing through the surgical portal apparatus. Sutures extending into a body cavity through a surgical portal apparatus may be used to, for example, temporarily retain tissue, manipulate tissue, anchor tissue or operate peripheral devices. In an attempt to reduce the number of incision sites required to complete a given surgical procedure, a single surgical portal apparatus may be used to pass one or more sutures into a body cavity, in addition to providing access for one or more devices. A single anchor device may have numerous suture ends that extend therefrom and through the surgical portal apparatus. The sutures extending through the surgical portal apparatus may become tangled as each is manipulated or as one or more instruments are inserted and withdrawn from the assembly. Also, a surgeon may confuse the suture ends during the course of a surgery. Tangling or confusion of the suture ends may unnecessarily complicate the procedure and increase time necessary to complete the procedure.

Therefore, it would be beneficial to have a suture management system for use with surgical portal apparatus.

### SUMMARY

Accordingly, a surgical portal apparatus for use in surgical procedures incorporating at least one suture is provided. The surgical portal apparatus includes a portal member defining a longitudinal axis and having a longitudinal opening therethrough for receiving a surgical object, and a suture retaining insert configured to be selectively received in the portal member. The suture retaining insert may include a base and a plurality of spaced projections extending radially outward from the base. The projections are radially spaced whereby adjacent projections define a suture receiving passage adapted to accommodate a suture.

The suture retaining insert may include at least three projections. The at least three projections are arranged to define at least two suture receiving slots between adjacent projections. The portal member may include an internal shelf adjacent the internal opening for supporting the suture retaining insert. The base of the insert may include an opening for receiving a surgical object therethrough. The base may be generally annular in configuration. The surgical portal apparatus may further include a seal member disposed along the longitudinal opening of the portal member for forming a substantial seal about the surgical object.

In another aspect, the surgical portal apparatus includes a portal member defining a longitudinal axis and having a longitudinal opening therethrough for receiving a surgical object and proximal and distal ends and a plurality of individual suture retaining members positioned adjacent the proximal end of the portal member. The suture retaining members each include inner portions defining a slot for receiving a suture extending though the longitudinal opening in the portal member. The suture retaining members may each be generally C-shaped.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
**FIG. 1** is a perspective side view of a surgical portal apparatus according to an embodiment of the present disclosure;
**FIG. 2** is a top view of the surgical portal apparatus of **FIG. 1****;**
**FIG. 3** is a top view of an alternate embodiment of a surgical portal apparatus according to the present disclosure;
**FIG. 4A** is a top view of an insert according to an alternate embodiment of the present disclosure;
**FIG. 4B** is a side views of the insert of **FIG. 4A****;**
**FIG. 5A** is a top view of an insert according to another embodiment of the present disclosure;
**FIG. 5B** is a side view of the insert of **FIG. 5A****;**
**FIG. 6** is a top view of a surgical portal apparatus according to another embodiment of the present disclosure;
**FIG. 7A** is a top view of a suture retainer of **FIG. 6****;**
**FIG. 7B** is a side view of the suture retainer of **FIG. 7A****;** and
**FIG. 8** is a top view of a surgical portal apparatus according to an alternate embodiment of the present disclosure.

### DETAILED DESCRIPTION

The suture management apparatus and systems herein disclosed may be configured for use in various surgical procedures, including laparoscopic, endoscopic, arthroscopic and orthopedic surgery. The suture management systems provides passage between a subject's body cavity and the outside atmosphere and is capable of receiving surgical instruments of various sizes and configurations. Embodiments of the presently disclosed suture management systems are configured to receive, for example, clip appliers, graspers, dissectors, retractors, staplers, laser probes, photographic devices, endoscopes and laparoscopes, tubes, and the like. Such instruments are collectively referred to herein as "instruments" or "instrumentation."

In addition to the instruments, the suture management systems also allows the passage of one or more sutures therethrough, e.g., during an arthroscopic procedure. When several sutures are introduced into the subject's body through the suture management system, the sutures might tangle with each other or be confused by a surgeon. Suture tangle and/or confusion may, at the very least, inconvenience the clinicians conducting the surgical procedure. To minimize the possibility of sutures tangling with one another or a surgeon from confusing the sutures, the suture management system incorporates a suture retaining member for retaining the one or more sutures.

Referring now to the drawings wherein like reference numerals illustrate similar components throughout the several views, there are illustrated embodiments of surgical portal apparatus according the principles of the present disclosure. As shown in the drawings and as described throughout the following description, as is traditional when referring to relative positioning on an object, the term "proximal" refers to the end of the apparatus which is closer to the user and the term "distal" refers to the end of the apparatus which is further from the user.

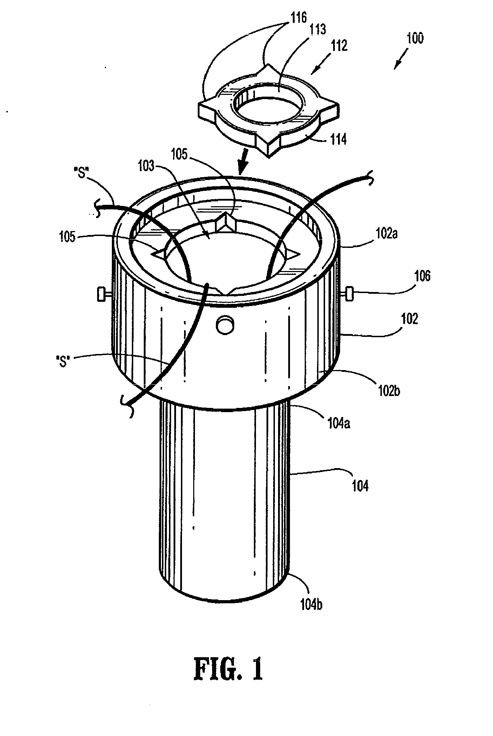

Referring initially to **FIG. 1**, an embodiment of a surgical portal apparatus according to the present disclosure is shown generally as surgical portal apparatus **100.** Surgical portal apparatus **100** includes a portal member **102,** a sleeve **104** extending distally from portal member **102,** and a suture management apparatus **110** configured to engage portal member **102.** Surgical portal apparatus **100** may be configured for use with any known endoscopic or laparoscopic instrument.

Portal member **102** defines a substantially cylindrical member having an open proximal end **102a,** a substantially open distal end **102b** and defining a passage **103** therebetween. Distal end **102b** of portal member **102** may be integrally formed with sleeve **104.** Alternatively, portal member **102** may be configured for selectable engagement with sleeve **104.** Portal member **102** may be constructed of plastic, polymer or other like material. Portal member **102** may be disposable, or in the alternative, reusable. Portal member **102** may be rigid, or alternatively, substantially flexible. Portal member **102** may include one or more anchors **106** or other suture securing means for securing one or more sutures **10** extending through surgical portal apparatus **100.** Portal member **102** may further include one or more seal members (not shown) having any seal arrangement for receiving an instrument in a sealed manner. As will be discussed in further detail below, proximal end **102a** of portal member **102** is configured to receive suture management apparatus **110.**

Sleeve **104** is configured to be inserted through the skin into a body cavity with the aid of an obturator (not shown), or may instead, include a blade or piercing tip for penetrating through the skin and into a body cavity. Sleeve **104** forms a substantially tubular member having proximal and distal ends **104a, 104b** and defining a first longitudinal passage **103** extending therebetween. Sleeve **104** may be composed of plastic, metal, polymers or the like. Sleeve **104** may be disposable, or in the alternative, reusable. Sleeve **104** may be rigid, or alternatively, sleeve **104** may be flexible. Sleeve **104** may be open, or instead, may be configured to include one or more seal members (not shown) along the length thereof. In an alternate embodiment, proximal end **104a** of sleeve **110** may be configured for operable engagement with suture management device **110.**

Suture management apparatus **110** defines a suture retaining insert **112** configured for operable engagement with portal member **102.** Insert **112** may be composed of plastic, polymer, metal or any other suitable material. Insert **112** includes a substantially annular base **114** and a plurality of projections or partitions **116** radial spaced thereabout. Annular base **114** defines an opening **113** configured for receipt of elongated objects therethrough. Opening **113** may be of varying diameters and may include a seal member (not shown) for receiving an instrument in a sealed manner. Projections **116** may be integrally formed with base **112.** Alternatively, projections **116** may be securely affixed to and/or selectively removable from base **114.** As shown, projections **116** include substantially triangular-shaped members; however, alternate configurations are envisioned.

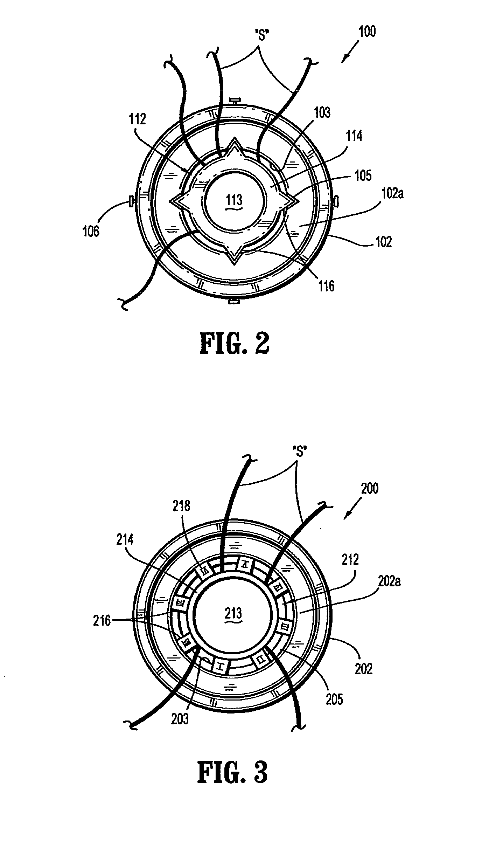

With reference still to **FIGS. 1** and **2****,** portal member **102** includes a plurality of cut-outs **105** configured to receive insert **112.** Cut-outs **105** are formed in proximal end **102a** of portal member **102** about passage **103.** Cut-outs **105** correspond in number and placement to projections **116** of insert **112.** Cut-outs **105** are configured to receive projections **116** of insert **112** as insert **112** is received in proximal end **102** of portal member **102.** As shown, cut-outs **105** are configured to loosely receive projections **116;** however, cut-outs **105** and/or projections **116** may be configured such that insert **112** frictionally engages proximal end **102a** of portal member **102.** In an alternate embodiment, cut-outs **105** may be configured to selectively receive projections **116** in a locking manner. For example, one or more of cut-outs **105** may include a lip (not shown) configured such that rotation of insert **112** once projections **116** are received in cut-outs 105 would cause a portion of projection **116** to engage the lip, thereby securing insert **112** within proximal end **102a** of portal member **102.** Rotation of insert **112** in an opposite direction would cause projections **116** to disengage the lip, thereby releasing insert **112** from portal member **102.**

In operation, surgical portal apparatus **100** operates in a manner similar to conventional access assemblies. Sleeve **104** is inserted through tissue, either with a piercing tip (not shown) or with the aid of an obturator (not shown). Once received through the tissue, surgical portal apparatus **100** may receive instruments through passage **103** in the absence of insert **112,** or instead, through opening **113** formed in insert **112.** Prior to receipt of one or more sutures "S" through surgical portal apparatus **100,** insert **112** is removed from passage **103.** One or more sutures "S" are then received through passage **103** of surgical portal apparatus **100** in any conventional manner. Sutures "S" are then extended radially outward from passage **103** over proximal end **102a** of portal member **102** between cut-outs **105** (**FIG. 1**). A surgeon may then place insert **112** within passage **103,** thereby retaining sutures "S" in an outer perimeter of passage **103** closer to portal member **102 (****FIG. 2****).** Once insert **112** is in place, elongated objects may be passed through opening **113** without causing the tangling of sutures "S". Insert **112** may be removed as necessary to add, remove and/or relocate sutures "S" within passage **103.**

Turning to **FIG. 3****,** an alternate embodiment of a suture management system of the present disclosure is shown generally as surgical portal apparatus **200.** Surgical portal apparatus **200** is substantially similar in form and function to surgical portal apparatus **100** described hereinabove, and therefore will only be described as relates to the differences therebetween. Surgical portal apparatus **200** includes a portal member **202** and an insert **212.** Insert **212** includes a plurality of projections **216** extending radially from annular base **214.** Annular base **214** defines an opening **213** for receipt of an elongated object. Projections **216** include markings **218** to assist in identifying one or more sutures "S" retained between projections **216.** Markings **218** may be letters, numbers, symbols, colors or other identifying feature. Alternatively, or in addition, markings (not shown) may be included on proximal end **202a** of portal member **202.** Insect **212** is configured to be received within a proximal end **202a** of portal member **202.** Proximal end **202a** includes a recessed portion or shelf **205.** Shelf **205** is configured to engaging projections **216** of insert **212** as insert **212** is received within passage **203.** The configuration of insert **212** and portal member **202** permits insert **212** to be rotated within passage **203** while maintaining one or more sutures "S" between projections **216** and away from opening **213.** In this manner, insert **212** operates to prevent sutures "S" from becoming tangled and/or confused. Insert **212** further permits selective relocation of sutures "S" about passage **103** without removing insert **212** from portal member **202.**

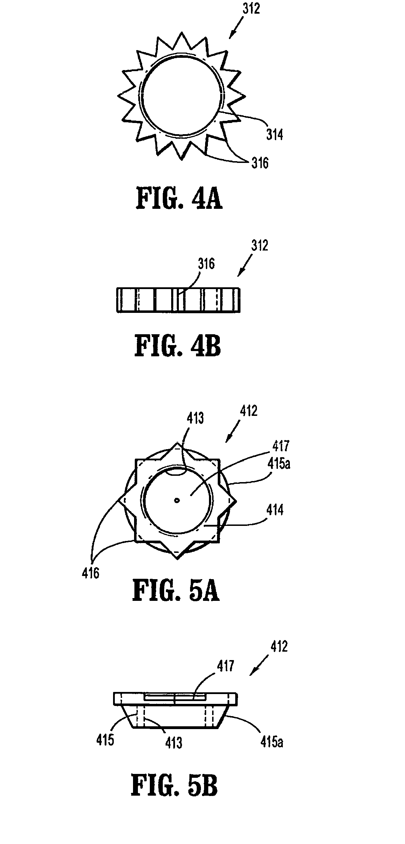

With reference now to **FIGS. 4A** and **4B****,** an alternate embodiment of a suture retaining insert according to the present disclosure is shown generally as suture retainer insert **312.** Insert **312** is substantially similar in form and function to inserts **112, 212** described hereinabove. Insert **312** includes a plurality of projections 316 extending from an annular base **314.** Projections **316** are configured to retain a single suture "S" (**FIG. 1**) therebetween. In this manner, sutures "S" may be maintained completely separate from one another. Portal members **102 (****FIG. 2****)** and **202 (****FIG. 3****)** may be configured to receive insert **312.**

Referring now to **FIGS. 5A** and **5B****,** another embodiment of a suture retaining insert according to the present disclosure is shown generally as suture retaining insert **412.** Insert **412** is substantially similar to inserts **112, 212, 312** described hereinabove. Insert **412** includes a plurality of projections **416** extending from an annular base **414.** Annular base **414** defines an opening **413** configured for receiving an elongate object therethrough. Insert **412** further includes a distal extension **415** extending from base **414** and a seal member formed in opening **413.** Distal extension **415** is sized and dimensioned to be received within passage **103, 203** of access assemblies **100, 200** such that projections **416** engage cut-outs **105** or shelf **205,** respectively. A tapered deformable coating **415a** extends about distal extension **415.** Deformable coating **415a** is configured to frictionally engage proximal end **102a, 202a** of portal member **102, 202,** respectively, as insert **412** is received within passage **103, 203,** respectively, thereby securely retaining suture "S" within passage **103, 203** against portal member **102, 202.** Furthermore, deformable coating **415a** creates a seal between portal member **102, 202** and insert **412** thereby preventing escape of insufflation gas through surgical portal apparatus **100, 200**, respectively. Seal member **417** permits an instrument to be received through insert **412** in a sealed manner.

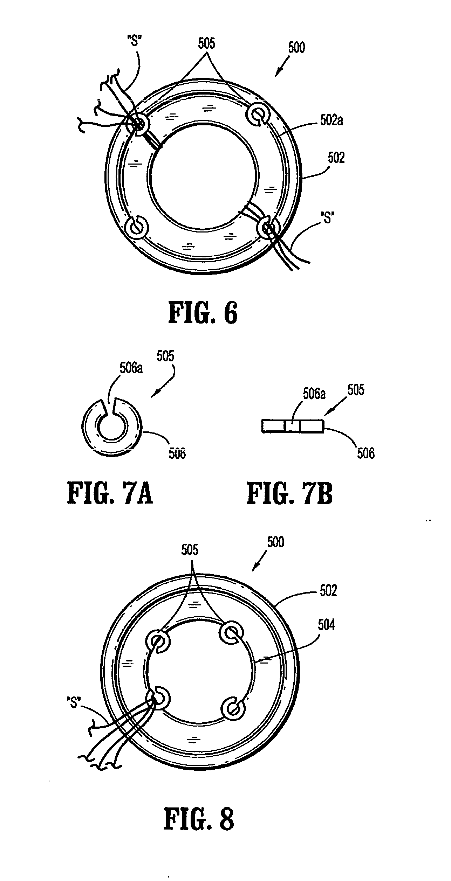

Turning now to **FIG. 6**, an alternate embodiment of a surgical portal apparatus according to the present disclosure is shown generally as surgical portal apparatus or surgical portal apparatus **500.** Surgical portal apparatus includes a portal member **502** defining a passage **503** configured for receipt of an elongated object. Positioned about a proximal end **502a** of portal member **502** is a plurality of suture retainers **505.** With particular reference to **FIGS. 7A** and **7B****,** suture retainers include a substantially flat annular base **506** including a slot **506a** for receiving one or more sutures "S". Suture retainers **505** maintain suture "S" away from passage **503,** thereby preventing an instrument (not shown) inserted through surgical portal apparatus **500** from engaging and causing the entangling of sutures "S". Suture retainers **505** may be integrally formed with, securely affixed to and/or removable attached to portal member **502.** In an alternate embodiment **(****FIG. 8****),** suture retainers are positioned on a proximal end of sleeve **504.**

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure.

## Claims

1. A surgical portal apparatus for use in surgical procedures incorporating at least one suture, which comprises:
a portal member defining a longitudinal axis and having a longitudinal opening therethrough for receiving a surgical object; and
a suture retaining insert configured to be selectively received in the portal member, the suture retaining insert including a base and a plurality of spaced projections extending radially outward from the base, the projections being radially spaced whereby adjacent projections define a suture receiving passage adapted to accommodate a suture.

2. The surgical portal apparatus according to claim 1, wherein the suture retaining insert includes at least three projections, the at least three projections being arranged to define at least two suture receiving slots between adjacent projections.

3. The surgical portal apparatus of claim 1 or claim 2, wherein the portal member includes an internal shelf adjacent the internal opening for supporting the suture retaining insert.

4. The surgical portal apparatus of any preceding claim, wherein the annular base of the insert includes an opening for receiving a surgical object therethrough.

5. The surgical portal apparatus of any preceding claim, wherein the base is generally annular in configuration.

6. The surgical portal apparatus of any preceding claim, further including a seal member disposed along the longitudinal opening of the portal member for forming a substantial seal about the surgical object.

7. A surgical portal apparatus, which comprises:
a portal member defining a longitudinal axis and having a longitudinal opening therethrough for receiving a surgical object and proximal and distal ends; and
a plurality of individual suture retaining members positioned adjacent the proximal end of the portal member, the suture retaining members each including inner portions defining a slot for receiving a suture extending though the longitudinal opening in the portal member.

8. The surgical portal apparatus of claim 9, wherein the suture retaining members are each generally C-shaped.
